# EUROPÄISCHE PATENTSCHRIFT

(11) **EP 3 017 760 B1**
(45) Veröffentlichungstag und Bekanntmachung des Hinweises auf die Patenterteilung: **19.06.2019**
(21) Anmeldenummer: 14198887.3
(22) Anmeldetag: 18.12.2014
(51) Int. Cl.: A61B 5/087, G01F 1/66

(54) **Atemrohr zum Einsatz in Ultraschall-Durchflussmesssystemen**
Breathing tube for use in ultrasound flow meter systems
Tube respiratoire destiné à être utilisé dans des systèmes de débitmètre à ultra-sons

(30) Priorität: 10.11.2014 DE 102014016608
(43) Veröffentlichungstag der Anmeldung: 11.05.2016
(73) Patentinhaber: ndd Medizintechnik AG, 8005 Zürich (CH)
(72) Erfinder: Buess, Christian, Dr., 8810 Horgen (CH); Kleinhappl, Erich, 8820 Wädenswil (CH); Sengel, Martin, 8200 Schaffhausen (CH)
(74) Vertreter: Maikowski & Ninnemann Patentanwälte Partnerschaft mbB

(56) Entgegenhaltungen:
- WO-A1-2014/137145
- DE-A1- 3 941 546
- DE-A1- 19 605 652
- DE-A1-102009 036 288
- JP-A- 2008 107 234
- KR-A- 20030 086 870
- US-A- 5 234 117
- US-A1- 2007 267 012
- US-B1- 6 612 306

## Beschreibung

Die Erfindung betrifft ein Atemrohr zum Einsatz in Ultraschall-Durchflussmesssystemen zur Bestimmung des Volumenstromes und/oder der Molmasse der Atmung von Menschen und Tieren.

Zur Messung der Atemströmung wird in der Regel eines der folgenden Messverfahren eingesetzt:
1. Verfahren, welche auf der Messung des durch die Strömung bedingten Druckabfalls über einen Widerstand im Atemrohr beruhen;
2. Verfahren, welche die Geschwindigkeit der Gase mittels Abkühlung eines geheizten Drahtes in der Strömung bestimmen;
3. Verfahren, welche auf der Messung der Rotationsgeschwindigkeit von Turbinen basieren und
4. Verfahren, welche die Laufzeiten von Ultraschallimpulsen messen, die mit und entgegen der Strömung laufen.

Das auf Ultraschall-Laufzeitmessung basierende Verfahren, das in der EP 0 597 060 A1 beschrieben ist, wird im medizinischen Umfeld seit einigen Jahren vermehrt ein- gesetzt. Die medizinische Anwendung unterscheidet sich von einer industriellen Anwendung durch folgende Besonderheiten.
- Es besteht ein sehr großer Messbereich mit hoher Ansprechgeschwindigkeit, d.h. sowohl sehr hohe Spitzenflüsse als auch sehr tiefe Flüsse müssen genau mit hoher Abtastrate gemessen werden.
- Es existieren stark wechselnde Gaszusammensetzungen. Bei gewissen medizinischen Testverfahren werden unterschiedliche Gase eingesetzt. Im Idealfall haben diese keinen Einfluss auf die Messung der Atemstromgeschwindigkeit
- Es müssen gute Hygieneeigenschaften bestehen. Das bedeutet, dass das Gerät beim Wechsel der Patienten reinigbar sein muss, um eine Kreuzkontamination unter allen Umständen zu vermeiden.

Bei Durchflussmessgeräten, welche auf Ultraschall-Laufzeitmessung basieren, werden zur Vermeidung von Kreuzkontamination häufig auswechselbare Atemrohre eingesetzt. In der EP 0 597 060 B1, der US 5,419,326 A und der US 5,647,370 A werden derartige auswechselbare Atemrohre beschrieben.

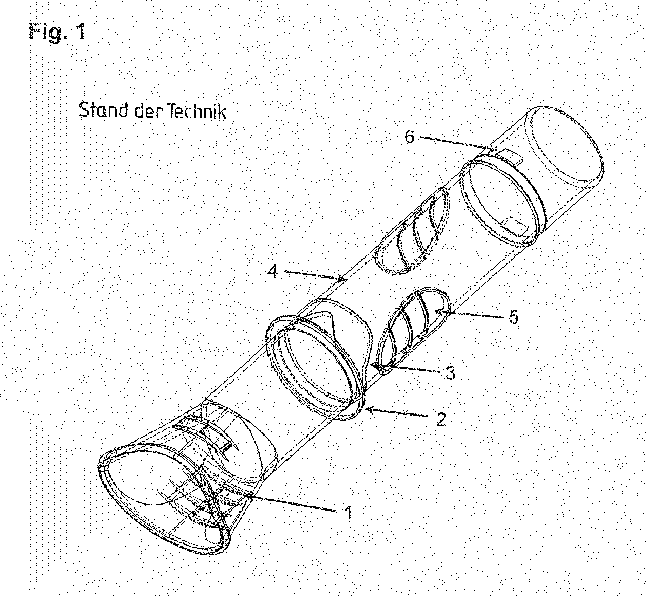

Diese bislang verwendeten Atemrohre weisen im Wesentlichen einen runden oder leicht ovalen Querschnitt auf. Oft wird ein Innendurchmesser von 20 mm bevorzugt, weil dieser einem verbreiteten Rohrdurchmesser in der Anästhesie entspricht. In der Figur 1 ist ein Ausführungsbeispiel eines Atemrohrs nach diesem Stand der Technik zur Verwendung in Spirometern zur Lungenfunktionsdiagnostik dargestellt. Das Atemrohr weist dabei folgende Elemente auf:
- Ein integriertes Mundstück 1, welches der Patient mit den Lippen umschließt,
- einen Ring 2, mit dreieckiger Positionsmarke, welche die Position des Atemrohres im Messsystem eindeutig festlegt,
- Dichtlippen 3, welche das Atemrohr gegenüber dem Messsystem abdichten,
- einen Körper 4 des Atemrohrs mit einem kreisrunden Innendurchmesser von ca. 20 mm,
- ovale Schallöffnungen 5, welche eine Ultraschallübertragung zwischen den beiden, schräg angeordneten, Sende- und Empfangselementen des hier nicht mehr dargestellten Ultraschall-Durchflussmesssystems erlaubt, und
- einen Widerhaken 6, welcher verhindert, dass das Atemrohr unabsichtlich wieder aus dem Messsystem herausgeschoben werden kann.

Die Atemrohre werden üblicherweise in Spritzgusstechnik hergestellt. Die Öffnungen der Schallöffnungen sind üblicherweise mit Netzen versehen, welche beim Produktionsprozess in das Material eingespritzt werden. Diese Netze weisen eine sehr kleine offene Fläche auf, erlauben eine Übertragung von Schallimpulsen und verhindern andererseits einen Strömungsabriss und somit starke Turbulenzen im Atemrohr. Da pro Patient nur ein Atemrohr verwendet wird, verhindern diese Netze, dass es zu Kreuzkontamination zwischen Patienten kommen kann.

Die vorbeschriebenen austauschbaren Atemrohre weisen aber aufgrund ihrer Konstruktionsweise einige Nachteile auf, die im Folgenden wiedergegeben sind:
- Der Ring mit der dreieckigen Positionsmarke garantiert nicht, dass das Atemrohr vom Benutzer immer in der exakt gleichen Position in das Messsystem eingeschoben wird.
- Das Atemrohr kann beispielsweise nicht vollständig eingeschoben oder auch nicht exakt ausgerichtet sein (unerwünschte Rotation des Atemrohrs). Ein nicht korrekt im Messsystem positioniertes Atemrohr führt aber zu Messfehlern in der Geschwindigkeitsmessung.
- Die Ultraschall-Durchstrahlung der Messstrecke in einem runden Querschnitt ist nicht optimal und erfasst die oberen bzw. unteren Anteile des Luftstromes nicht. Bei ungleicher Verteilung des Luftstromes über den Querschnitt kann dies ebenfalls zu Messfehlern führen.

Die US 6,612,306 B1 beschreibt ein Gerät zur Messung des Stickoxidgehalts in der Atemluft eines Individuums. Dieses Gerät zeichnet sich durch ein spezifisches Einwegventil aus.

Die JP 2008 107234 A beschreibt ein Ultraschallmessgerät, bei dem ein Ultraschallmessröhrchen durch seitlich angeordnete Vorsprünge besonders fest und sicher in einem Gehäuse gehalten wird.

Die DE 10 2009 036 288 A1 beschreibt eine Vorrichtung zur Entnahme einer Gasprobe aus der menschlichen Atmung mit einem auswechselbaren Atemrohr. Das Atemrohr weist dabei eine Aussparung als Eingriffsöffnung für eine Absaugleitung auf. Durch die Absaugleitung kann eine Gasprobe aus dem Atemrohr entnommen werden. Ferner ist ein Eingriffsabschnitt mit spezifischer Ausgestaltung vorgesehen, der mit der Aussparung zusammenwirkt.

Die DE 196 05 652 A1 beschreibt ein Verfahren zur Kalibrierung eines Durchflussmessers, bei dem die Geometrie eines Messrohrs erfasst und anhand der ermittelten geometrischen Größen ein Kalibrierfaktor berechnet wird.

Aufgabe der Erfindung ist es, ein gattungsgemäßes Atemrohr derart weiterzubilden, dass die oben erläuterten Nachteile behoben werden. Insbesondere soll im System erkannt werden, ob ein Atemrohr in seiner korrekten Position eingesetzt ist.

Erfindungsgemäß wird diese Aufgabe durch die Kombination der Merkmale des Anspruchs 1 gelöst. Beim gattungsgemäßen Atemrohr zum Einsatz in Ultraschall-Durchflussmesssystemen zur Bestimmung des Volumenstroms und /oder der Molmasse der Atmung von Menschen und Tieren wird demzufolge das Atemrohr zumindest bereichsweise mit einem mehreckigen Querschnitt ausgeführt, wobei Öffnungen im Atemrohr, die zur Durchleitung von Ultraschallimpulsen dienen, über ein gewebeartiges Netz verschlossen sind. Dabei weist das Atemrohr mindestens einen Indikator auf, der über ein externes optisches Mittel auslesbar ist.

Erfindungsgemäß ist der mindestens eine Indikator durch die Formgebung der äußeren Oberfläche des Atemrohres gebildet. Dabei ist die äußere Oberfläche des Atemrohres zur Bildung des mindestens einen Indikators im Bereich mindestens einer Kante kammartig geformt.

Der zumindest bereichsweise vorgesehene mehreckige Querschnitt des Atemrohrs verhindert, dass das Atemrohr verdreht in das Messsystem eingesetzt werden kann. Vorzugsweise ist der mehreckige Querschnitt hierzu asymmetrisch ausgeführt, um ein falsches Einsetzen des Atemrohres (beispielsweise durch versehentliches Verdrehen um einen bestimmten Winkelbetrag) zu verhindern.

Über den mindestens einen Indikator kann automatisch das Vorhandensein des Atemrohrs im Messsystem festgestellt werden. Darüber hinaus kann auch die korrekte Positionierung in Längsachse des Atemrohrs innerhalb des Messsystems sichergestellt werden. Über die Auswahl des Indikators kann zusätzlich eine Information zum Typ des Atemrohrs ausgelesen werden.

Vorteilhafte Ausgestaltungen der Erfindung ergeben sich aus den sich an den Hauptanspruch anschließenden Unteransprüchen.

Der mindestens eine Indikator kann über ein optisches Mittel, wie beispielsweise eine Lichtschranke oder eine Reflexionslichtschranke ausgelesen werden. Diese Lichtschranken sind in dem entsprechenden Ultraschall-Durchflussmesssystem integriert, in welchem das Atemrohr eingesetzt wird.

Erfindungsgemäß ist der mindestens eine Indikator durch die Formgebung der äußeren Oberfläche des Atemrohrs gebildet. Dabei ist das Atemrohr zur Bildung des mindestens einen Indikators im Bereich mindestens einer Kante kammartig geformt. Durch die hierdurch gebildeten Zwischenräume in der kammartig geformten Kantenoberfläche können beispielsweise Lichtstrahlen auf die optischen Sensoren hindurchtreten.

Gemäß einer vorteilhaften Ausgestaltung der Erfindung weist das Atemrohr einen nahezu rechteckigen Querschnitt auf, wobei die Außenseiten des Atemrohrs leicht angeschrägt verlaufen. Hiermit kann verhindert werden, dass das Atemrohr versehentlich um 180° versetzt in das Messsystem eingesetzt wird.

Vorteilhaft weist das Atemrohr auf seiner Oberfläche mindestens eine umlaufende Dichtlippe auf. Dabei kann die mindestens eine Dichtlippe im Zusammenwirken mit der Messvorrichtung, in welche das Atemrohr einsetzbar ist, aufgrund resultierender gleichmäßiger nach außen wirkender Kraft eine sichere Abdichtung mit der Innenfläche der Messvorrichtung, d.h. des Ultraschall-Durchflussmesssystems, bewirken.

Die Öffnungen im Atemrohr, die zur Durchleitung der Ultraschallimpulse dienen, sind über ein gewebeartiges Netz verschlossen. Das gewebeartige Netz kann dabei jeweils beim Spritzvorgang zur Erzeugung des aus einem spritzbaren Kunststoff bestehenden Atemrohrs aus dem gleichen Material mitspritzbar sein. Alternativ kann das gewebeartige Netz aber auch aus einem zu dem Atemrohr unterschiedlichen Material bestehen und in einem gesonderten Arbeitsschritt zur Abdeckung der Öffnung mit dem Atemrohr verbindbar sein.

Gemäß einer weiteren vorteilhaften Ausgestaltung der Erfindung kann das Atemrohr ein separat aufsteckbares Mundstück aufweisen.

Des Weiteren kann das Atemrohr zusätzlich einen Filter aufweisen, der vorzugsweise auch aufsteckbar gestaltet werden kann.

Schließlich kann das Atemrohr auch auf der äußeren Oberfläche mindestens einen Vorsprung aufweisen, über den das Atemrohr aus dem Ultraschall-Durchflussmesssystem ausschiebbar ist.

Vorteilhaft können auch unterschiedliche Typen des Atemrohres unterschiedliche innere Querschnitte aufweisen, um damit auf spezielle Anwendung bezüglich des Volumens des Atemrohres und der Messauflösung optimiert zu werden.

Eine zuverlässige Erkennung des Typs des Atemrohrs über den wenigstens einen Indikator ermöglicht zusätzlich vorteilhaft eine Anpassung der Messauswertung des Ultraschall-Durchflussmesssystems. Hier kann die Linearisierung des gemessenen Flusssignals, d.h. der mathematische Zusammenhang zwischen dem gemessenen Transitzeiten und der ausgegebenen Geschwindigkeit des Volumenstroms, auf das jeweils eingesetzte Atemrohr abgestimmt werden.

Weitere Merkmale, Einzelheiten und Vorteile der Erfindung werden anhand von in der Zeichnung dargestellten Ausführungsbeispielen näher erläutert.

Es zeigen:
- Figur 1:: eine schematische Darstellung eines Atemrohrs nach dem Stand der Technik;
- Figur 2:: eine perspektivische Ansicht einer ersten Ausführungsform des erfindungsgemäßen Atemrohrs;
- Figur 3:: das Atemrohr gemäß Figur 2 in Seiten-, Drauf- und Frontsicht;
- Figur 4:: eine schematische Darstellung zur Verdeutlichung der Durchstrahlung der Messstrecke bei einem runden und rechteckigen Atemrohr;
- Figur 5:: einen Querschnitt durch das Atemrohr gemäß Figur 2 und
- Figur 6:: einen Längsschnitt durch ein Atemrohr gemäß einer zweiten Ausführungsform der Erfindung.

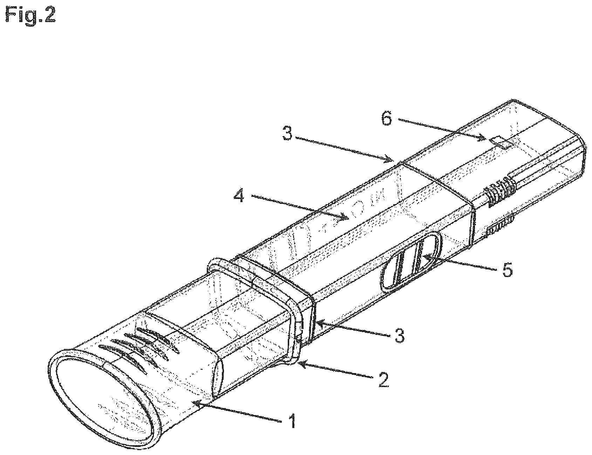

Eine Ausführungsform des erfindungsgemäßen Atemrohrs ist in Figur 2 in perspektivischer Ansicht dargestellt. Dieses Atemrohr dient zum Einsetzen in ein hier nicht näher dargestelltes Ultraschall-Durchflussmessgerät, wie es beispielsweise aus der EP 0 597 060 A1 bekannt ist. Dieses Atemrohr ist für den Einmalgebrauch bei einem Patienten ausgelegt und verfügt über mechanische Indikatoren, welche über ein optisches System erkannt werden können. In der perspektivischen Ansicht gemäß Figur 2 ist zu erkennen, dass das Atemrohr ein integriertes Mundstück 1 aufweist. Ein entsprechender Anschlagring 2 mit Auswurflasche definiert die Endposition beim Einschieben in das hier nicht näher dargestellte Messsystem und ermöglicht andererseits mit Hilfe der Auswurflasche ein leichtes Herausschieben des Atemrohrs nach Verwendung desselben.

Das Atemrohr weist zwei umlaufende Dichtlippen 3 in seinem vorderen und hinteren Bereich auf, welche zur Abdichtung gegenüber dem hier nicht dargestellten Ultraschall-Messsystem dienen. Der eigentliche Körper 4 des Atemrohres hat einen annähernd rechteckigen Querschnitt, wobei die Seitenwandungen allerdings leicht angeschrägt sind. Somit ergibt sich genau genommen eine Trapezform mit abgerundeten Ecken. Die beiden vorgesehenen Öffnungen auf den gegenüberliegenden Seiten des Atemrohres, die zur Übertragung des Ultraschalls dienen, sind jeweils mit Netzen verschlossen. Schließlich ist ein Widerhaken 6 am Atemrohr angespritzt, welcher verhindert, dass das Atemrohr unabsichtlich wieder aus dem Messsystem herausgeschoben werden kann.

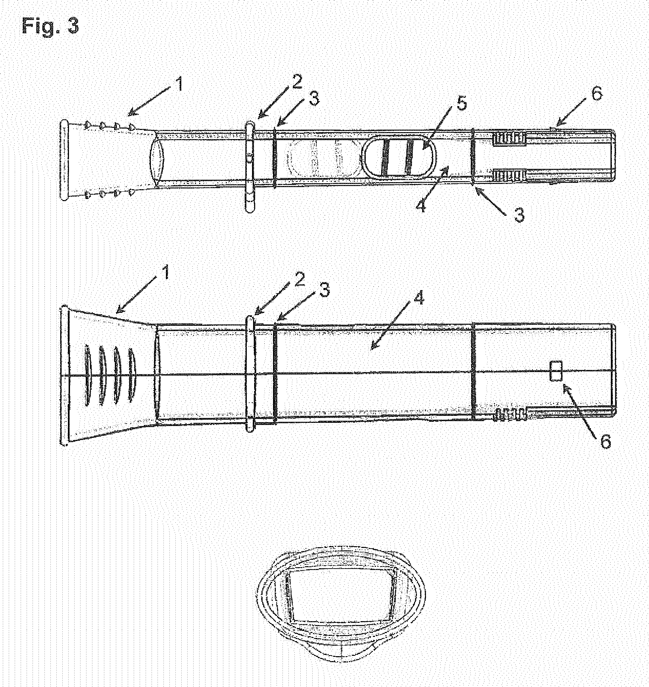

Im oberen und unteren Randbereich des Atemrohrs sind auf der äußeren Oberfläche jeweils in der Kante zwei mechanische Indikatoren in Form einer angespritzten kammartigen Struktur vorgesehen. Die Figur 3 zeigt die zuvor beschriebene Realisierungsvariante zusätzlich in einer Seiten-, Drauf- und Frontansicht Die Frontansicht verdeutlicht den im Wesentlichen rechteckigen Querschnitt des Atemrohrs. Es ist ebenfalls ersichtlich, dass die Seitenwände durch ihre Abschrägung so gestaltet sind, dass das Atemrohr nicht um 180° gedreht in das Messsystem eingesetzt werden kann.

Gegenüber den bisher üblichen Atemrohren zur Verwendung in Ultraschall-Durchflussmesssystemen weist das Atemrohr der vorliegenden Erfindung in dem hier dargestellten Ausführungsbeispiel einen nahezu rechteckigen Querschnitt auf. Diese Form weist gegenüber einer runden oder leicht ovalen Querschnittsform eine bessere "Durchdringung" des Querschnitts durch den Ultraschallstrahl auf.

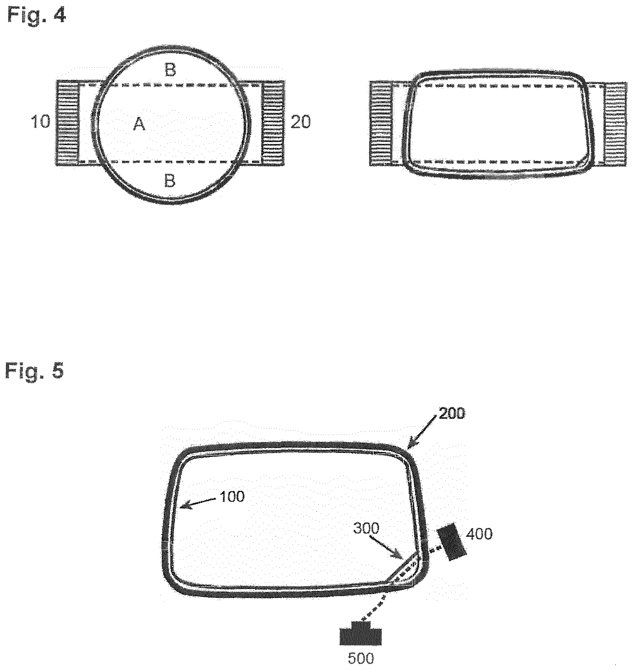

In der Figur 4 sind zwei Varianten eines Atemrohrs zusammen mit den seitlich angeordneten Ultraschallzellen dargestellt. Die Ultraschallzellen sind mit 10 bzw. 20 bezeichnet. Bei beiden handelt es sich sowohl um Sender als Empfänger von Ultraschallsignalen. Die Fläche zwischen den beiden unterbrochenen Linien A zwischen den Ultraschallzellen stellt den vom Ultraschallstrahl abgedeckten Bereich des Volumenstroms dar. Das bedeutet, dass in diesem Bereich der Volumenstrom im Atemrohr durch den Ultraschallstrahl gemessen werden kann. Der gesamte Volumenstrom im äußeren Bereich B wird dagegen nicht durch den Ultraschall erfasst. Bei ungleichmäßiger Verteilung des Volumenstroms über den Querschnitt des Atemrohrs führt dieser Effekt zu Fehlern im Messergebnis. Im Falle des rechteckartigen Querschnitts des Atemrohrs ist das Verhältnis der Flächen A zu B besser, d.h. ein größerer Prozentsatz des Volumenstroms wird tatsächlich vom Ultraschallmesssignal erfasst. Dies wird also bei der rechten Ausführungsform in Figur 4, die im Wesentlichen der Form der vorliegenden erfindungsgemäßen Ausführung entspricht, zu einer Verkleinerung des Fehlers bei ungleichmäßiger Verteilung des Volumenstroms im Atemrohr führen.

Die Figur 5 zeigt eine Realisierungsvariante der optischen Detektion der mechanischen Indikatoren des Atemrohrs 100, welches in einem Ultraschall- Durchflussmesssystem, das hier mit 200 bezeichnet ist, eingeführt ist. In der Darstellung weist das Atemrohr eine kammartige Struktur 300, die in Längsrichtung ausgerichtet ist, im rechten unteren Bereich des Atemrohrs auf. Die kammartige Struktur ist grundsätzlich bereits in Figur 2 in der perspektivischen Darstellung gezeigt. Über eine Lichtquelle 400, beispielsweise eine LED-Quelle, wird ein Lichtstrahl über den kammartigen Indikator zur einem Zeilensensor 5 geleitet. Der Atemrohrhalter des Messsystems 200 muss zu diesem Zweck im Bereich des Indikators durchsichtig ausgestaltet sein. Die Lichtquelle und der zugehörige Strahlen- gang müssen so gestaltet sein, dass die kammartige Struktur vollständig auf dem Zeilensensors abgebildet wird. Durch Auslesen der Informationen des Zeilensensors können unterschiedliche Informationen erhalten und im System verarbeitet werden:
a) das Vorhandensein eines Atemrohrs,
b) die Positionen des Atemrohrs innerhalb des Messsystems sowie
c) der Typ des Atemrohrs.

Die Position des Atemrohrs kann erfasst werden, da die Indikatoren entlang der Längsachse angeordnet sind. Zur Erkennung des Atemrohrtyps, wird dieser in die Struktur des kammartigen Indikators kodiert. Erhöhungen und Vertiefungen in der mechanischen Struktur des Indikators erzeugen charakteristische Licht-/Schattenabfolgen auf dem Zeilensensors. Aus diesem Abbild des Indikators auf dem Zeilensensors kann in der Steuerung des Ultraschall-Durchflussmesssystems eine Identifikationsnummer berechnet werden.

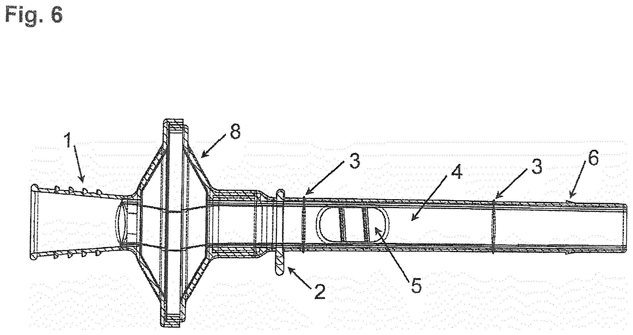

Die Figur 6 zeigt einen Längsschnitt durch eine alternative Ausführungsvariante des erfindungsgemäßen Atemrohrs. Grundsätzlich entspricht dieses Atemrohr demjenigen gemäß Figur 2. Allerdings ist hier im Unterschied zu der in Figur 2 dargestellten Ausführungsvariante das Mundstück 1 und ein zusätzlicher Filtereinsatz 8 aufsteckbar mit dem Restkorpus 4 des Atemrohrs verbunden. Die hier vorgesehene Steckverbindung ermöglicht ein einfaches Austauschen von Mundstück 1 und/oder Filtereinsatz 8.

## Patentansprüche

1. Atemrohr zum Einsatz in Ultraschall-Durchflussmesssystemen zur Bestimmung des Volumenstromes und/oder der Molmasse der Atmung von Menschen und Tieren, wobei das Atemrohr zumindest bereichsweise einen mehreckigen Querschnitt aufweist, wobei Öffnungen im Atemrohr, die zur Durchleitung von Ultraschallimpulsen dienen, über ein gewebeartiges Netz verschlossen sind und wobei am Atemrohr mindestens ein Indikator ausgebildet ist, der über ein externes optisches Mittel auslesbar ist,
**dadurch gekennzeichnet,**
**dass** der mindestens eine Indikator durch die Formgebung der äußeren Oberfläche des Atemrohres gebildet ist und dass die äußere Oberfläche des Atemrohres zur Bildung des mindestens einen Indikators im Bereich mindestens einer Kante kammartig geformt ist.

2. Atemrohr nach Anspruch 1, **dadurch gekennzeichnet, dass** es einen nahezu rechteckigen Querschnitt aufweist, wobei die Außenseiten des Atemrohres leicht angeschrägt verlaufen.

3. Atemrohr nach Anspruch 1 oder 2, **dadurch gekennzeichnet, dass** es auf seiner äußeren Oberfläche mindestens eine umlaufende Dichtlippe aufweist.

4. Atemrohr nach Anspruch 3, **dadurch gekennzeichnet, dass** die Oberfläche des Atemrohrs eine derart gewölbte Form aufweist, dass die Dichtlippen im Zusammenwirken mit der Messvorrichtung, in welche es einsetzbar ist, aufgrund einer resultierenden gleichmäßigen nach außen wirkenden Kraft eine sichere Abdichtung bewirken.

5. Atemrohr nach einem der vorherigen Ansprüche, **dadurch gekennzeichnet, dass** das gewebeartige Netz jeweils beim Spritzvorgang zur Erzeugung des aus einem spritzbaren Kunststoff bestehenden Atemrohrs aus dem gleichen Material mitspritzbar ist.

6. Atemrohr nach einem der Ansprüche 1 bis 4, **dadurch gekennzeichnet, dass** das gewebeartige Netz aus einem zu dem Atemrohr unterschiedlichen Material besteht.

7. Atemrohr nach einem der vorherigen Ansprüche, **dadurch gekennzeichnet, dass** das Atemrohr ein separates aufsteckbares Mundstück aufweist.

8. Atemrohr nach einem der vorherigen Ansprüche, **dadurch gekennzeichnet, dass** es ein Filter aufweist, das vorzugsweise aufsteckbar ist.

9. Atemrohr nach einem der vorherigen Ansprüche, **dadurch gekennzeichnet, dass** es auf der äußeren Oberfläche mindestens einen Vorsprung aufweist, über den das Atemrohr aus dem Ultraschall-Durchflussmesssystem ausschiebbar ist.

## Claims

1. Breathing tube for use in ultrasound flow meter systems for determining the volumetric flow and/or the molar mass of the respiration of humans and animals, wherein the breathing tube has at least in part a polygonal cross section, wherein openings in the breathing tube, which serve for the passage of ultrasound pulses, are closed by a fabric-like net, and wherein at least one indicator is formed on the breathing tube and is readable via an external optical means,
**characterized in that**
the at least one indicator is formed by the shaping of the outer surface of the breathing tube, and **in that** the outer surface of the breathing tube is shaped like a comb in the region of at least one edge in order to form the at least one indicator.

2. Breathing tube according to Claim 1, **characterized in that** it has an almost rectangular cross section, wherein the outer sides of the breathing tube extend with a slight bevel.

3. Breathing tube according to Claim 1 or 2, **characterized in that** it has at least one circumferential sealing lip on its outer surface.

4. Breathing tube according to Claim 3, **characterized in that** the surface of the breathing tube has a shape curved in such a way that, in cooperation with the measuring device into which the breathing tube is insertable, the sealing lips provide a secure seal by virtue of a resulting uniform, outwardly acting force.

5. Breathing tube according to any of the preceding claims, **characterized in that**, during the injection-moulding operation for producing the breathing tube made of an injectable plastic, the fabric-like net is injectable from the same material.

6. Breathing tube according to any of Claims 1 to 4, **characterized in that** the fabric-like net is made of a material different than the breathing tube.

7. Breathing tube according to any of the preceding claims, **characterized in that** the breathing tube has a separate mouthpiece that can be plugged on.

8. Breathing tube according to any of the preceding claims, **characterized in that** it has a filter, which preferably can be plugged on.

9. Breathing tube according to any of the preceding claims, **characterized in that** it has at least one projection on the outer surface, via which projection the breathing tube can be pushed out of the ultrasound flow meter system.

## Revendications

1. Tube respiratoire destiné à être utilisé dans des systèmes de débitmètres à ultrasons pour déterminer le débit volumique et/ou la masse molaire de la respiration humaine et animale, dans lequel le tube respiratoire présente au moins par zones une section transversale polygonale, dans lequel des ouvertures présentes dans le tube respiratoire, qui servent à la transmission d'impulsions ultrasonores, sont fermées au moyen d'un filet de type tissu, et dans lequel au moins un indicateur, qui peut être lu par un moyen optique externe, est réalisé sur le tube respiratoire, **caractérisé en ce que** ledit au moins un indicateur est réalisé par mise en forme de la surface extérieure du tube respiratoire
et **en ce que** la surface extérieure du tube respiratoire est réalisée sous forme de peigne afin de réaliser ledit au moins un indicateur dans la région d'au moins un bord.

2. Tube respiratoire selon la revendication 1, **caractérisé en ce qu'**il présente une section transversale pratiquement rectangulaire, dans lequel les faces extérieures du tube respiratoire sont légèrement biseautées.

3. Tube respiratoire selon la revendication 1 ou 2, **caractérisé en ce qu'**il présente au moins une lèvre d'étanchéité périphérique sur sa surface extérieure.

4. Tube respiratoire selon la revendication 3, **caractérisé en ce que** la surface du tube respiratoire présente une forme incurvée telle que les lèvres d'étanchéité, en association avec le dispositif de mesure dans lequel il peut être inséré, assurent une bonne étanchéité grâce à la force uniforme ainsi produite et s'exerçant vers l'extérieur.

5. Tube respiratoire selon l'une des revendications précédentes, **caractérisé en ce que** le filet de type tissu peut être respectivement coinjecté à partir du même matériau lors du processus d'injection destiné à produire le tube respiratoire constitué d'une matière plastique injectable.

6. Tube respiratoire selon l'une des revendications 1 à 4, **caractérisé en ce que** le filet de type tissu est constitué d'un matériau différent de celui du tube respiratoire.

7. Tube respiratoire selon l'une des revendications précédentes, **caractérisé en ce que** le tube respiratoire comporte un embout buccal séparé pouvant être emboîté.

8. Tube respiratoire selon l'une des revendications précédentes, **caractérisé en ce qu'**il comprend un filtre qui peut de préférence être emboîté.

9. Tube respiratoire selon l'une des revendications précédentes, **caractérisé en ce qu'**il présente sur sa surface extérieure au moins une saillie par l'intermédiaire de laquelle le tube respiratoire peut être poussé hors du système de débitmètre à ultrasons.
